# EUROPEAN PATENT SPECIFICATION

(11) **EP 2 817 011 B1**
(45) Date of publication and mention of the grant of the patent: **05.01.2022**
(21) Application number: 13751188.7
(22) Date of filing: 21.02.2013
(51) Int. Cl.: A61K 31/664, A61K 31/675, A61K 31/7068, A61P 35/00, A61K 31/685

(54) **TREATMENT OF CANCER**
KREBSBEHANDLUNG
TRAITEMENT DU CANCER

(30) Priority: 21.02.2012 US 201261601438 P; 24.01.2013 US 201361756419 P
(43) Date of publication of application: 31.12.2014
(73) Proprietor: ImmunoGenesis, Inc., Houston TX 77010 (US)
(72) Inventor: KROLL, Stewart, Oakland, CA 94705 (US)
(74) Representative: Ricker, Mathias
(86) International application number: PCT/US2013/027091
(87) International publication number: WO 2013/126539

(56) References cited:
- WO-A1-2008/083101
- WO-A1-2010/048330
- WO-A2-2007/002931
- WO-A2-2012/009288
- WO-A2-2012/135757
- WO-A2-2012/142520
- Borad et al.: "TH-302, A TUMOR-SELECTIVE HYPOXIA-ACTIVATED PRODRUG, COMPLEMENTS THE CLINICAL BENEFITS OF GEMCITABINE IN FIRST LINE PANCREATIC CANCER", thresholdpharm.com , January 2011 (2011-01), XP002741175, Retrieved from the Internet: URL:http://www.thresholdpharm.com/pdf/bora d_asco_gi_1-11.pdf [retrieved on 2015-06-18]
- Denise T. Powell: "Threshold Pharmaceuticals' TH-302 Continues to Demonstrate Promising Activity in Pancreatic Cancer Phase 1/2 Clinical TrialPhase 2 Controlled Trial in Pancreatic Cancer Enrollment on Track", investor.thresholdpharm.com , 24 January 2011 (2011-01-24), XP002741176, Retrieved from the Internet: URL:http://investor.thresholdpharm.com/rel easedetail.cfm?releaseid=545135 [retrieved on 2015-06-18]
- MITESH BORAD ET AL: "Complete Phase 1B Study of TH-302 in Combination with Gemcitabine, Docetaxel or Pemetrexed", EUROPEAN SOCIETY FOR MEDICAL ONCOLOGY (ESMO) ANNUAL MEETING, 1 October 2010 (2010-10-01), XP055143265,
- None

## Description

### FIELD OF THE INVENTION

This invention provides a method as defined in claim 1 and a combination for use as defined in claim 2.

The invention relates generally to the fields of medicine, pharmacology, and medicinal chemistry.

### BACKGROUND OF THE INVENTION

Cancer is one of the major causes of human morbidity and mortality. Cancer treatment is challenging because it is often difficult to kill cancer cells without damaging or killing normal cells. Damaging or killing normal cells during cancer treatment causes adverse side effects in patients and can limit the amount of anticancer drug administered to a cancer patient. It is also difficult to kill cancer cells in regions distant from the vasculature where anticancer drugs fail to penetrate.

Many cancer cells are more hypoxic than normal cells. Tumor hypoxia is associated with resistance to anticancer therapies, cancer relapse, and poor prognosis. Certain drugs in preclinical and clinical development target hypoxic cancer cells. These drugs, called hypoxia-activated prodrugs or "HAPs", are administered in an inactive or prodrug form, but are activated and become toxic in a hypoxic environment. PCT Pat. Pub. Nos. WO 07/002931 and WO 08/083101 describe HAPs such as TH-302, known by the chemical name (2-bromoethyl)({[(2-bromoethyl)ammo][(2-nitro-3-metbylimidazol-4-yl)methoxy]phosphoryl})-amine. See Duan et al., 2008, "Potent and highly selective hypoxia-activated achiral phosphoramidate mustards as anticancer drugs," J Med. Chem 51: 2412.

### SUMMARY OF THE INVENTION

This invention provides a method of identifying a subject as eligible for treatment of its cancer with a combination of a hypoxia activated prodrug TH-302 or TH-281 or a pharmaceutically acceptable salt thereof; in combination with a chemotherapeutic agent that is a nucleoside analog, wherein the method is as defined in claim 1.

This invention further provides a combination of a hypoxia activated prodrug TH-302 or TH-281 or a pharmaceutically acceptable salt therof; and a chemotherapeutic agent that is a nucleoside analog for use in the treatment of cancer in a subject having characteristics (i) and (ii) as defined in claim 2.

### BRIEF DESCRIPTION OF THE DRAWINGS

**Fig. 1** shows the mechanism by which TH-302 converts to a chemotoxic agent under hypoxic conditions in vivo at or around the hypoxic tumor site.
**Fig. 2A** is a Kaplan Meyer curve showing the increase in progression free survival (PFS) in human subjects having pancreatic cancer undergoing treatment in a clinical trial. The control group treated with gemcitabine alone is compared with the two groups that received gemcitabine plus TH-302 at 240 mg/m² or 340 mg/m² respectively. **Fig 2B** shows that the overall survival (OS) trends towards an improvement by about two months.
**Fig. 3A** is a waterfall plot showing the decrease in tumor size in the three groups. The groups receiving TH-302 followed by gemcitabine showed more tumor shrinkage. **Fig. 3B** is a waterfall plot showing the change in the level of tumor marker CA19-9 in blood. The group showing the fastest decrease had been administered TH-302 at 340 mg/m² followed by gemcitabine for each of three weeks in the 4-week cycle.
**Fig. 4** shows a study design for a human clinical trial of TH-302 and gemcitabine in patients with previously untreated advanced pancreatic cancer.
**Fig. 5A** and **Fig 5B** show the increase in PFS and overall survival, respectively, following treatment with gemcitabine alone, TH-302 at 240 mg/m² followed by gemcitabine, or TH-302 at 340 mg/m² followed by gemcitabine.
**Fig. 6** shows a subgroup analysis of PFS according to patients' baseline demographics and disease characteristics. The effectiveness of the gemcitabine plus TH-302 treatment combination associates with certain such characteristics, as described below,
**Fig. 7** shows the tumor shrinkage observed in all subjects grouped according to the treatment protocol received.

### DETAILED DESCRIPTION

This detailed description of the invention is divided into sections for the convenience of the reader. Section I provides definitions of certain terms used herein. Section II provides general information about hypoxia activated prodrugs and nucleoside chemotherapeutic drugs useful in the implementation of this invention. Section III describes pharmaceutical formulations of TH-302 and TH-281 as illustrative of the hypoxia-activated prodrug class. Section IV describes chemotherapeutics that are nucleoside analogs, exemplified by gemcitabine. Section V generally describes methods for treating cancer using such drugs. Section VI describes clinical testing methodology. Section VII describes treatment of patent subgroups. Section VIII describes management of adverse events. The general description is followed by three examples that provide illustrative embodiments of the methods and compositions provided by the invention.

### I. Definitions

The following definitions are provided to assist the reader. Unless otherwise defined, all terms of art, notations, and other scientific or medical terms or terminology used herein are intended to have the meanings commonly understood by those of skill in the chemical and medical arts. In some cases, terms with commonly understood meanings are defined herein for clarity and/or for ready reference, and the inclusion of such definitions herein should not be construed as inconsistent with the definition of the term as generally understood in the art.

"A," "an," and, "the" include plural referents unless the context clearly dictates otherwise. Thus, for example, reference to a compound refers to one or more compounds or at least one compound. As such, the terms "a" (or "an"), "one or more", and "at least one" are used interchangeably herein.

"About" as used herein is used to provide flexibility to a numerical range endpoint by providing that a given value may be "a little above" or "a little below" the endpoint accounting for variations one might see in measurements taken among different instruments, samples, and sample preparations. In one aspect, "about" refers to ± 20% of a quantity and includes, but is not limited to, ± 15%, ± 10%, and ± 5% of the quantity.

"Active agent" refers to a compound with a desired pharmacological effect and includes all pharmaceutically acceptable forms of the active agent described. For example, the active agent can be in an isomeric mixture, a solid complex bound to an ion exchange resin, or the like. In addition, the active agent can be in a solvated form, Active agent also includes all pharmaceutically acceptable salts, derivatives, and analogs of the active agent being described, as well as combinations thereof. For example, the pharmaceutically acceptable salts of the active agent may include, without limitation, the sodium, potassium, calcium, magnesium, ammonium, tromethamine, L-tysine, L-arginine, N-ethylglucamine, N-methylglucamine and salt forms thereof, as well as combinations thereof and the like. Any form of the active agent may be suitable for use in the compositions of the invention, *e*.*g*., a pharmaceutically acceptable salt of the active agent, a free acid or free base of the active agent, or a mixture thereof.

"Administering" or "administration of" a drug to a patient (and grammatical equivalents of this phrase) refers to direct administration, which may be administration to a patient by a medical professional or may be self-administration, and/or indirect administration, which may be the act of prescribing a drug. For example, a physician or clinic that instructs a patient to self-administer a drug and/or provides a patient with a prescription for a drug is administering the drug to the patient.

"Advanced solid tumor" refers to a solid tumor that has relapsed, progressed, metastasized after, and/or is refractory to, the initial or first line treatment. Advanced solid tumors include, but are not limited to, metastatic tumors in bone, brain, liver, lungs, lymph node, pancreas, prostate, and soft tissue (sarcoma).

"Cancer" refers to leukemias, lymphomas, carcinomas, and other malignant tumors of potentially unlimited growth that can expand locally by invasion and systemically by metastasis, Examples of cancers include, but are not limited to, cancer of the adrenal gland, bone, brain, breast, bronchi, colon and/or rectum, gallbladder, head and neck, kidneys, larynx, liver, lung, neural tissue, pancreas, prostate, parathyroid, skin, stomach, and thyroid. Certain other examples of cancers include, acute and chronic lymphocytic and granulocytic tumors, adenocarcinoma, adenoma, basal cell carcinoma, cervical dysplasia and in situ carcinoma, Ewing's sarcoma, epidermoid carcinomas, giant cell tumor, glioblastoma multiforma, hairy-cell tumor, intestinal ganglioneuroma, hyperplastic corneal nerve tumor, islet cell carcinoma, Kaposi's sarcoma, leiomyoma, leukemias, lymphomas, malignant carcinoid, malignant melanomas, malignant hypercalcemia, marfanoid habitus tumor, medullary carcinoma, metastatic skin carcinoma, mucosal neuroma, myeloma, mycosis fungoides, neuroblastoma, osteo sarcoma, osteogenic and other sarcoma, ovarian tumor, pheochromocytoma, polycythemia vera, primary brain tumor, small-cell lung tumor, squamous cell carcinoma of both ulcerating and papillary type, hyperplasia, seminoma, soft tissue sarcoma, retinoblastoma, rhabdomyosarcoma, renal cell tumor, topical skin lesion, veticulum cell sarcoma, and Wilm's tumor.

A "chemotherapeutic agent" is a pharmaceutical compound that is given to a cancer patient primarily to eradicate, diminish, stabilize, or decrease the growth rate or metabolism of one or more malignant tumors in the patient.

"Dose" and "dosage" refer to a specific amount of active or therapeutic agent(s) for administration.

"Dosage form" refers to physically discrete units suitable as unitary dosages for human subjects and other mammals, each unit containing a predetermined quantity of active agent calculated to produce the desired onset, tolerability, and therapeutic effects, in association with one or more suitable pharmaceutical excipients such as carriers.

"Excipient" includes any inert substance combined with an active agent such as TH-302 to prepare a convenient dosage form and vehicle for delivering the active agent.

"Formulation" and "composition" are used interchangeably and refer to a mixture of two or more compounds, elements, or molecules. In some aspects the terms "formulation" and "composition" may be used to refer to a mixture of one or more active agents with a carrier or other excipients. A pharmaceutical formulation is suitable for administration to a human or mammal.

"Hypoxia activated prodrug" or "HAP" refers to a prodrug wherein the prodrug is less active or inactive, relative to the corresponding drug, and comprises the drug and one or more bioreducible groups. HAPs include prodrugs that are activated by a variety of reducing agents and reducing enzymes, including without limitation single electron transferring enzymes (such as cytochrome P450 reductases) and two electron transferring (or hydride transferring) enzymes. In some embodiments, HAPs are 2-nitroimidazole triggered hypoxia-activated prodrugs. Examples of HAPs include, without limitation, TH-302, TH-281, PR-104 and AQ4N. Methods of synthesizing TH-302 are described in PCT Pat. App. Pub. Nos. WO 07/002931 and WO 08/083101. Methods of synthesizing PR104 are described in US Pat. App. No. 2007/0032455. Other examples of HAPs are described, for example, in US Pat. App. Nos. 2005/0256191, 2007/0032455 and 2009/0136521 and PCT Pat. App. Pub. Nos. WO 00/064864, WO 05/087075, and WO 07/002931.

"Patient" and "subject" are used interchangeably to refer to a mammal in need of treatment for cancer or other hyperproliferative disease. Generally, the patient is a human. Generally, the patient is a human diagnosed with cancer. In certain embodiments a "patient" or "subject" may refer to a non-human mammal such as a non-human primate, a dog, cat, rabbit, pig, mouse or rat such as animals used in screening, characterizing, and evaluating drugs and therapies.

"Pharmaceutically acceptable carrier, excipient, or diluent" refers to a carrier, excipient, or diluent that is useful in preparing a pharmaceutical formulation that is generally safe, non-toxic, and neither biologically nor otherwise undesirable, and includes a carrier, excipient, or diluent that is acceptable for human pharmaceutical use and/or veterinary use. A "pharmaceutically acceptable carrier, excipient, or diluent" can refer to one or more than one such carrier, excipient, or diluent.

"Pharmaceutically acceptable salt" refers to salts of active agents that are prepared with relatively nontoxic acids. The compound of the present invention contains relatively basic functionalities, and acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogen-phosphoric, sulfuric, monohydrogensulfuric, hydriodic, or phosphorous acids and the like, as well as the salts derived from relatively nontoxic organic acids like acetic, propionic, isobulyric, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, *e.g.*, Berge, S.M. et al., "Pharmaceutical Salts," Journal of Pharmaceutical Science, 66:1 19, 1977). Certain specific compounds of the present invention contain both basic and acidic functionalities that allow the compounds to be converted into either base or acid addition salts. The neutral forms of the compounds may be regenerated by contacting the salt with a base and isolating the parent compound in the conventional manner. The parent form of the compound differs from the various salt forms in certain physical properties, such as solubility in polar solvents, but otherwise the salts are equivalent to the parent form of the compound for the purposes of the present invention.

"Prodrug" refers to a compound that, after administration, is metabolized or otherwise converted to a biologically active or more active compound (or drug) with respect to at least one property. A prodrug, relative to the drug, is modified chemically in a manner that renders it, relative to the drug, less active or inactive, but the chemical modification is such that the corresponding drug is generated by metabolic or other biological processes after the prodrug is administered. A prodrug may have, relative to the active drug, altered metabolic stability or transport characteristics, fewer side effects or lower toxicity, or improved flavor (for example, see Nogrady, 1985, Medicinal Chemistry A Biochemical Approach, Oxford University Press, New York, pages 388-392). A prodrug may be synthesized using reactants other than the corresponding drug.

"Reduction" of a symptom or symptoms (and grammatical equivalents of this phrase) refers to decreasing the severity or frequency of the symptom(s), or elimination of the symptom(s).

A "side effect" is a sign, symptom, or laboratory finding that is attributable to an administered drug or therapy but is peripheral, secondary, or different from the intended effect of the drug or therapy on the target disease or condition. The side effect may be substantially benign, or it may be unwanted or adverse (an "adverse event", AE). It may be attributed to the drug or therapy (for example) for historical reasons, mechanistic reasons, because it occurs more frequently in a treated population than in a matched control population, or because the effect is reversible upon withdrawal of the drug or therapy.

A "solid tumor" is a malignancy that forms a discrete tumor mass: for example, cancer of brain, the breast, prostate, colorectum, kidney; sarcoma; melanoma, in contrast to lymphoproliferative malignancies (such as leukemia and lymphoma) which may diffusely infiltrate a tissue without forming a mass.

"Therapeutically effective amount" of a drug refers to an amount of a drug that, when administered to a patient with cancer or other hyperproliferative disease, will have the intended therapeutic effect, *e*.*g*., alleviation, amelioration, palliation or elimination of one or more manifestations of cancer or another hypaproliferalive disease in the patient. A therapeutic effect does not necessarily occur by administration of one dose, and may occur only after administration of a series of doses. Thus, a therapeutically effective amount may be administered in one or more administrations.

"Treating" or "treatment of a condition or patient refers to taking steps to obtain beneficial or desired results, including clinical results. For purposes of this invention, beneficial or desired clinical results include, but are not limited to, alleviation or amelioration of one or more symptoms of cancer or other hyperproliferative disease; diminishment of extent of disease; delay or slowing of disease progression; amelioration, palliation, or stabilization of the disease state; management of one or more side effects of another drug, or other beneficial results. Treatment of cancer may, in some cases, result in partial response or stable disease.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary.

Concentrations, amounts, and other numerical data may be expressed or presented herein in a range format, it is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 to about 5" should be interpreted to include not only the explicitly recited values of about 1 to about 5, but also include individual values and sub-ranges within the indicated range. Thus, included in this numerical range are individual values such as 2, 3, and 4 and sub-ranges such as from 1-3, from 2-4, and from 3-5, etc., as well as 1, 2, 3, 4, and 5, individually. This same principle applies to ranges reciting only one numerical value as a minimum or a maximum. Furthermore, such an interpretation should apply regardless of the breadth of the range or the characteristics being described.

### II. Hypoxia activated prodrugs

Embodiments of the present disclosure (not part of the claimed invention) may be conducted with hypoxia activated prodrugs having the structure shown in Formula I: 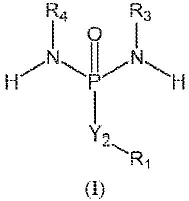 wherein Y₂ is O, S, NR₆, NCOR₆, or NSO₂R₆ wherein R₆ is C₁-C₆ alkyl, C₁-C₆ heteroalkyl, aryl, or heteroaryl; R₃ and R₄ are independently selected from the group consisting of 2-haloalkyl, 2-alkylsulfonyloxyalkyl, 2-heteroaikylsulfonyloxyalkyt, 2-arylsulfonyloxyalkyl, and 2-heteroalkylsulfonyloxyalkyl; R₁ has the formula L-Z₃; L is C(Z₁)₂; each Z₁ independently is hydrogen, halogen, C₁-C₆ alkyl, C₁-C₆ heteroalkyl, aryl, heteroaryl, C₃-C₈ cycloalkyl, heteroclyclyl, C₁-C₆ acyl, C₁-C₆ heteroacyl, aroyl, or heteroaroyl; or L is: 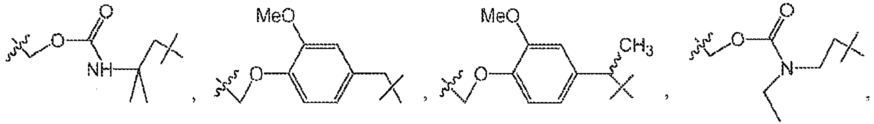 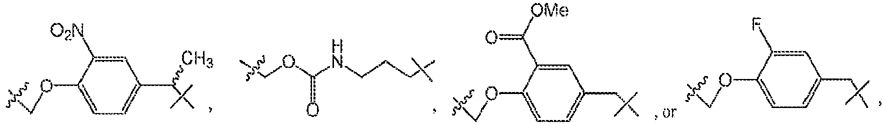 Z₃ is a bioreductive group having a formula selected from the group consisting of: 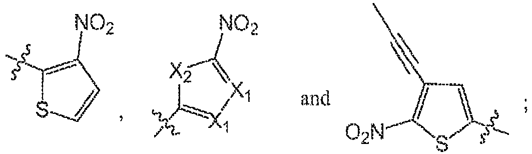 wherein each X₁ is independently N or CR₈; X₂ is NR₇, S, or O; each R₇ is independently C₁-C₆ alkyl, C₁-C₆ heteroalkyl, C₃-C₈ cycloalkyl, heterocyclyl, aryl or heteroaryl; and R₈ is independently hydrogen, halogen, cyano, CHF₂, CF₃, CO₂H, amino, C₁-C₆ alkyl, C₁-C₆ heteroalkyl, C₁-C₆ cycloalkyl, C₁-C₆ alkoxy, C₁-C₆ alkylatnino, C₁-C₆ dialkylamino, aryl, CON(R₇)₂, C₁-C₆ acyl, C₁-C₆ heteroacyl, aroyl or heteroaroyl; or a pharmaceutically acceptable salt thereof, Particular examples are TH-302 and TH-281. In the present invention, the hypoxia activated prodrug of Formula I is TH-302 or TH-281, or a pharmaceutically acceptable salt thereof.

"TH302" or "TH-302" refers to the compound having the following formula: 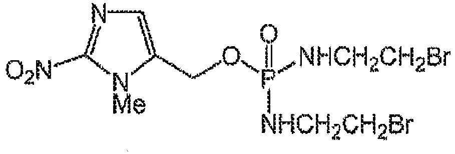 and includes pharmaceutically acceptable salts thereof.

"TH281" or "TH-281" refers to the compound having the following formula: 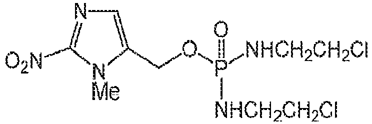 and includes pharmaceutically acceptable salts thereof.

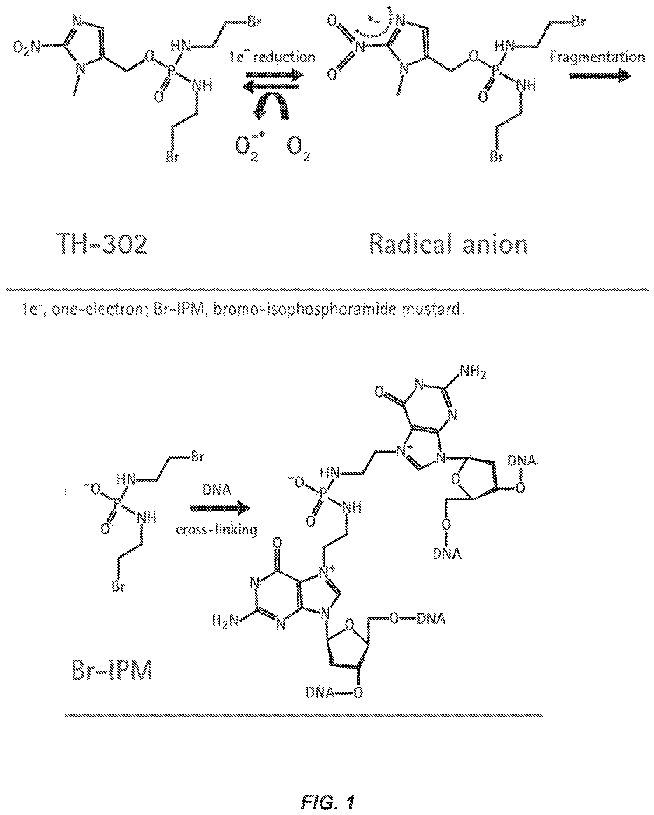

Fig. 1 shows the mechanism by which TH-302 converts to a chemotoxic agent under hypoxic conditions in vivo at or around hypoxic tumor sites.

When used in the general description , reference to the compounds TH-302 and TH-281 are for illustrative purposes for the general class of compounds having the structure shown in Formula I. Unless expressly limited to a particular compound, the various aspects discussed in reference to TH-302 or TH-281 may be put into practice using TH-302, TH-281, or other hypoxia activated prodrugs having the structure of Formula I, at the user's discretion. However, only embodiments relating to TH-302 or TH-281 are part of the claimed invention.

### III. Pharmaceutical formulations of TH-302 and TH-281

TH-302 and TH-281 can be administered to patients in accordance with the invention in any pharmaceutically acceptable formulation. For example. PCT Publications WO 08/083101 and WO 07/002931 disclose methods for preparing liquid pharmaceutical formulations of TH-302 and TH-281. WO 07/002931 discloses that TH-302 can be provided as a lyophilized powder in a vial and reconstituted in saline or 5% dextrose in water (D5W) immediately prior to administration. After reconstitution in D5W, the TH-302 formulation is used within 8 hours. The shelf life for this lyophilized TH-302 formulation is about 1 year at 2 to 8°C and longer at -20°C. WO 08/083101 discloses that TH-302 can be administered as a liquid formulation in ethanol (containing up to 50 mg of TH-302 per mL).

As described in PCT Pub. No. WO2010/048330 the poor aqueous solubility of the nitro-heteroaryl phosphoramide class of hypoxia-activated cancer drugs, such as TH-302, can be improved by providing a nonionic surfactant for prolonged storage in an alcohol environment. Formulations containing ethanol and a nonionic surfactant such as TWEEN 80^{®} can therefore be advantageously used for administering TH-302 or TH-281. TWEEN 80 is sorbitan mono-oleate polyoxyethylene, CAS number 9005-65-6. Advantageously, these preferred parenterally administrable pharmaceutical compositions provide improved stability and reduced degradation and precipitation for TH-302, as well as for other nitro-heteroaryl phosphoramide class hypoxia-activated cancer drugs with poor solubility, including TH-281. In addition, these formulations provide for a higher concentration of the active drug, *e*.*g*., TH-302, relative to other formulations that have been described. Because these liquid formulations are stable over the long term, no lyophilization of the drug is required, which eliminates the need to reconstitute a lyophilizate before use. This pharmaceutically acceptable formulation is suitable for parenteral administration.

Typically, the liquid compositions of the invention comprise from about 10 mg/mL to about 300 mg/mL of the active agent. One skilled in the art understands that the foregoing concentrations can be adjusted depending upon the particular active agent utilized and the amount of active agent desired in the final formulation. The amount of TH-302 included in the present liquid formulation is dictated by the intended use. Generally, the concentration of TH-302 will be in the range of 10 mg/mL to about 300 mg/mL or 30 mg/mL to about 300 mg/mL, more typically 50 mg/mL to 200 mg/mL, more usually 50 mg/mL to about 150 mg/mL, and even more usually 50 mg/mL to 125 mg/mL, and most usually greater than 50 mg/mL, such as about 60 mg/mL, 60 mg/mL to 100 mg/mL, 100 mg/mL to 150 mg/mL, 100 mg/mL to 200 mg/mL, or 100 mg/mL to about 300 mg/mL. These concentrations refer to the free base form of TH-302 or TH-281.

According to one embodiment, the carrier is ethanol, and the pharmaceutical formulation includes at least 5% v/v TWEEN 80^{®}. In one embodiment, the formulation comprises about 5 to 10% (v/v) TWEEN 80, 90-95% (v/v) ethanol, and about 50 mg/mL to 125 mg/mL TH-302 or TH-281, such as about 60 mg/mL TH-302. in one embodiment, the formulation comprises about 5% TWEEN 80, about 95% ethanol, and about 60 mg/mL TH-302.

Thus, in an embodiment, the active agent in the formulation is TH-302, including all pharmacologically acceptable forms. In one aspect, the TH-302 is administered as a liquid composition, wherein the TH-302 in its non-salt form. In other embodiments, the active agent is TH-281 in its non-salt form. In the embodiments of the invention, the active agent can be provided for dissolution into the formulation of the invention in any suitable form. For example, it can be in the form of a powder, pellet, or a granule (*i*.*e*., an aggregate of smaller units of active agent). Any pharmaceutical grade of TH-302 or TH-281 may be used.

In some embodiments, the formulation may optionally further comprise other components described herein. Thus, in various embodiments of the pharmaceutical formulations of the invention, mixtures of carriers are used. When a second carrier (in addition to ethanol) is used, it is generally N,N-dimethylacelamide (DMA). When used, the amide carrier is preferably included in an amount up to about 20% of the formulation by volume. Preferably the amount of amide carrier used is about 10% to about 20% by volume. In some embodiments the formulation consists of ethanol and TH-302. In some embodiments the formulation consists of ethanol, DMA and TH-302.

The compositions of the present invention can additionally include an antioxidant, preserving agents such as methyl-, ethyl-, and propyl-hydroxy-benzoates, butylated hydroxytoluene, and butylated hydroxyanisole; opacifying agents and chelating agents. Suitable antioxidants and chelating agents, include for example, butylated hydroxyanisole (BHA), butylated hydroxytoluene (BHT), propyl gallate (PG), ascorbyl palmitate, disodium EDTA (ethylenediamine tetraacetic acid; also known as disodium edentate), EDTA, tartaric acid, citric acid, citric acid monohydrate, and sodium sulfite. In one embodiment, the foregoing compounds are included in the pharmaceutical formulation in amounts in the range of about 0.01% to about 5% w/w. In one specific embodiment, the pharmaceutical formulation includes BHA, BHT, or PG used at a range of about 0.02% to about 1% and disodium EDTA, citric acid, or citric acid monohydrate used at a range of about 2% to about 5%. In one embodiment, the pharmaceutical formulation includes BHA used at about 0.05% w/w.

Any suitable method can be used to mix the formulation comprising the active agent, alcohol carrier, and non-ionic surfactant. In one embodiment, the active agent, alcohol carrier, and non-ionic surfactant are combined, and the mixture is administered directly to the patient, optionally after dilution.

The pharmaceutical formulations of the invention can be packaged in any packaging that facilitates stability of the drug formulation. The pharmaceutical formulations provided by this invention may be contained in a sterilized vessel such as syringes, vials, or ampoules of various sizes and capacities. The sterilized vessel may be of a non-DEHP material when using formulations that contain TWEEN and/or DMA. The sterilized vessel may optionally contain between 1-50 mL, 1-25 mL, 1-20 mL, 1-10 mL or 1-5 mL of the formulation. Sterilized vessels maintain sterility of the pharmaceutical formulations, facilitate transportation and storage, and allow administration of the pharmaceutical formulations without prior sterilization step.

The pharmaceutical formulation provided in vessels may be in a form that is suitable for direct administration or may be in a concentrated form that requires dilution relative to what is administered to the patient. For example, pharmaceutical formulation may be in a form that is suitable for direct administration via intravenous administration or may be in a concentrated form that is diluted prior to administration. In one embodiment, about 500 to 2000 mg of TH-302 in a formulation of the invention is administered to a patient over 30-60 minutes after dilution in D5W or saline to about 500 mL total volume.

The compositions of the present invention are useful in therapeutic applications, *e*.*g*., for treating cancer. While the formulations of this invention may be delivered via various routes of administration, they are typically administered intravenously (*e*.*g*., by infusion) but any acceptable method may be used (*e*.*g*., intraarterially, via local delivery by catheter or stent, and the like).

In one embodiment, a hypoxia-activated cancer drug formulation is infused through a connector, such as a Y site connector, that has three arms, each connected to a tube. As an example, BAXTER^{®} Y-connectors of various sizes can be used. A vessel containing hypoxia-activated cancer drug formulation is attached to a tube further attached to one arm of the connector. Infusion fluids, such as 0.9% sodium chloride, or 5% dextrose, or 5% glucose, or Lactated Ringer's, are infused through a tube attached to the other arm of the Y-site connector. The infusion fluids and hypoxia-activated cancer drug formulations are mixed inside the Y site connector. The resulting mixture is infused into the patient through a tube connected to the third arm of the Y site connector. The advantage of this administration approach over the prior art is that the hypoxia-activated cancer drug is mixed with infusion fluids before it enters the patient's body, thus reducing the time when decomposition of therapeutic formulations may occur due to contact with water. In some embodiments, the TH-302 or TH-281 is mixed less than 10, 5, 2 or 1 minutes before entering the patient's body. In some embodiments the TH-302 or TH-281 is mixed less than 8, 6, 4, 2 or 1 hours before entering the patient's body.

As noted above, pharmaceutical formulations according to the present invention provide the further advantage because the nonionic surfactant/alcohol solution can be readily mixed with water, D5W or saline, the formulations can be easily and readily further diluted just prior to administration. For example, the pharmaceutical formulations can be diluted with water, saline or D5W within the 8 hour period preceding administration to a patient.

In one embodiment, the infusion administration is performed after determining the mg dose for a patient by multiplying the patient's body surface area (in m²) by the prescribed TH-302 dose of either 240 mg/m² or 340 mg/m². The appropriate number of vials (for example, 100 mg/vial) of TH-302 are removed from a -20°C freezer and left in an ambient room condition for 30-60 minutes to allow vials to warm to room temperature. Each 100 mg vial is reconstituted with 25 mL sterile D5W or saline and shaken well. The number of mL of reconstituted TH-302 required is calculated by multiplying the desired mg dose by 0.25 (*e*.*g*., a 1000 mg dose requires 250 mL). Prior to adding reconstituted TH-302 to a 500/1000 mL sterile D5W (or saline) IV bag, the equivalent volume of TH-302 to be added to the bag is removed, so that when the reconstituted drug is added to the bag the total volume is 500/1000 mL.

Patients may be infused with TH-302 or TH-281 drug formulations for any therapeutically suitable time, *e*.*g*., about 15, 30, or 45 minutes or for 1, 2, 3, 4, 5 or more hours. The speed and volume of the infusion can be regulated according to the patient's needs. The regulation of the infusion of hypoxia-activated cancer drug formulations can be performed according to existing protocols. For illustration, a dose of <1000 mg may be diluted in 500 mL and infused over about 30 min; a dose of 1000 or higher may be diluted in 1000 mL and infused over about 60 min. Alternatively a dose may be diluted to a concentration of 1 to 4 mg/mL (e.g. 2 mg/mL) with the infusion duration dependent on the infusion volume. Dilution volumes and infusion times are based on total dose administered for TH-302 (longer infusion times are permitted based on physician judgment of the time required to administer the infusion volume; smaller infusion volumes are permitted based on the physician judgment of the tolerability of infusion volume).

An individual patient's surface area can be determined using routine methods known to oncologists and other medical service providers. For an adult human, a dose of 1 mg/m² of an active agent (drug) = about 1.7 mg of that agent or drug per patient (*i*.*e*., the prototypical adult human has 1,7 m² of surface area). Therefore, for example, 100 mg/m² of a drug = about 170 mg of that drug per patient.

As described in more detail below, the pharmaceutical formulations of the invention are co-administered with other chemotherapeutic agents, such as gemcitabine. Co-administration in the context of this invention is defined to mean the administration of more than one therapeutic agent in the course of a coordinated treatment to achieve an improved clinical outcome. Such co-administration may also be coextensive, that is, occurring during overlapping periods of time. The additional agent administered may be in any conventional form and may include infusion fluids, therapeutic compounds, nutritious fluids, anti-microbial fluids, buffering and stabilizing agents. Therapeutic compounds, in this context, include, but are not limited to, anti-neoplastic agents, alkylating agents, agents that are members of the retinoid superfamily, antibiotic agents, hormonal agents, plant-derived agents, biologic agents, interleukins, interferons, cytokines, immunomodulating agents, and monoclonal antibodies. As discussed in detail below, when the additional agent is an anti-neoplastic drug and both drugs are administered on the same day, administration of the two agents is sometimes contemporaneous but not simultaneous, with the hypoxia-activated prodrug administered first, and the second drug administered at least 30 minutes later.

### IV. Nucleoside derivatives

Chemotherapeutic agents that are used in combination with the hypoxia activated prodrug are nucleoside analogs. These are designed to be incorporated into DNA synthesis during the process of mitosis, but thereafter to prevent lengthening of the replicating DNA. They may also irreversibly bind and inhibit ribonucleotide reductase (RNR), thus preventing or decreasing synthesis of deoxynucleotides.

Included are chemotherapeutics having the structure shown in Formula II: 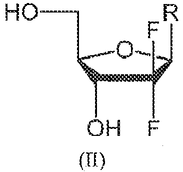 where R is a nitrogen-containing monocyclic or heterocyclic aromatic compound chosen so that the compound may mimic a nucleoside and block enzymatic reactions associated with DNA biosynthesis. R may be selected from the following: 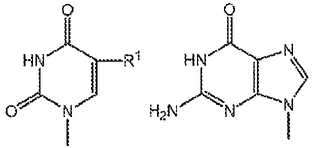 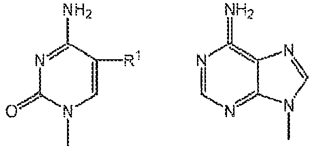 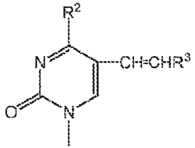 where R₁ is hydrogen, methyl, bromo, fluoro, chloro or iodo; and R₂ is hydroxy; R₃ is hydrogen, bromo, chloro or iodo. See U.S. Patent 4,808,614.

Exemplary is the compound gemcitabine (distributed under the trade name Gemzar^{®}), which has the following structure:

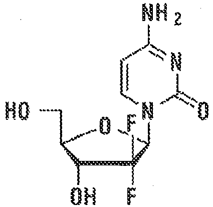

When used in the general description of this invention, reference to the compound gemcitabine is for illustrative purposes for the general class of compounds having the structure shown in Formula II. Unless expressly limited to a particular compound, the various aspects of the invention discussed in reference to gemcitabine may be put into practice using gemcitabine or other nucleoside analogs with chemotherapeutic activity having the structure of Formula II, at the user's discretion.

### V. Administration of TH-302 and TH-281 to treat cancer

TH-302 (or TH-281 or other compound of Formula I) can be administered as monotherapy, *i.e.,* alone, not in combination with any other anticancer agent, to treat cancer. However, this is not part of the claimed invention.

TH-302 (or TH-281 or other compound of Formula I) is usually administered intravenously (*e*.*g*., by infusion) for monotherapy or combination therapy. In various embodiments, a TH-302/ethanol/TWEEN 80/ DMA formulation as discussed *supra* is diluted into D5W or saline for infusion. Although a variety of dosage schedules are possible, typically TH-302 is administered intravenously (*e*.*g*., by infusion) for one or more cycles of (a) once weekly for 3 consecutive weeks followed by a week of no TH-302 administration (*e*.*g*., administered on days 1, 8, and 15 of a 28 day cycle) referred to as a 4 week cycle; (b) once per week; (c) once every three weeks; or (d) twice every three weeks (*e*.*g*., administered on days 1 and 8 of a 21-day cycle). For a dosing regimen comprising a 4 week cycle, TH-302 can be administered, for example, at a dose of 240 mg/m² or 340 mg/m², and these doses can be employed for weekly administration and once every three week administration, as well. Other doses may be selected based on the patient's age, health and other factors.

Like other anticancer agents, TH-302 and TH-281 are usually administered in multiple cycles. For example and not for limitation, TH-302 may be administered using a 4 week cycle for from 1 to 13 cycles, from 1 to 7 cycles, or from 1 to 4 cycles. As a second example, TH-302 may be administered at a frequency of once every week for 3 to 52, 3 to 28, 3 to 16, or 3 to 8 weeks. As a third example, TH-302 may be administered at a frequency of once every three weeks for 3 to 52, 3 to 28, 3 to 16, or 3 to 8 weeks. It will be recognized by medical professionals that certain of these periods of TH-302 administration include one or more weeks of drug holidays during which no TH-302 is administered.

The above described doses and dosing frequencies are also useful in the methods of treating cancer, including but not limited to pancreatic cancer, provided by the invention that comprise administering TH-302 or TH-281 to cancer patients in combination with administering another anticancer drug, such as gemcitabine. In these combination therapy methods, the therapeutic efficacy of the combination treatment is maximized and the toxicity of the combination treatment minimized by administering the TH-302 or TH-281 and the other anticancer drug non-contemporaneously. Administering two drugs a certain time period apart in accordance with the present invention is referred to as "non-contemporaneous administration" of the two drugs. Administering two drugs together or one immediately after the other (with) no or less than a 30 minute delay between ceasing the administration of the first and initiating the administration of the second drug) is referred to as "contemporaneous administration" of the two drugs,

Thus, described herein is a method of treating cancer by administering two anticancer drugs, a hypoxia activated prodrug that is either TH-302 or TH-281 and an anticancer drug that is not hypoxically activated, such as gemcitabine, to a patient in need of such treatment, wherein the second drug is administered at least about 30 minutes after the immediate prior administration of the hypoxia activated prodrug is stopped. In an embodiment, the second drug is administered 30 minutes to 8 hours after administration of the first drug has stopped. In other embodiments, the second drug is administered 1 to 6 hours after administration of the first drug has stopped (*e.g.,* about 2 hours, about 3 hours or about 4 hours after administration of the HAP drug). In some embodiments, the duration of the time between administration of TH-302 or TH-281 and the non-HAP drug (*e.g.,* gemcitabine) is from 1 to 10 hours, from 2 to 6 hours, or from 3 to 5 hours. In exemplary embodiments, the non-HAP anticancer drug is administered 1 hour or longer (*e.g.,* 1, 2, 4, or 6 hours) after administration of the HAP has stopped. Typically, at least a 30 minute to 2 hour delay is employed. Thus, generally, the time of administration of the non-HAP drug is at least 30 minutes to one hour, at least 2 hours, at least 4 hours, and sometimes 24 hours or more after administration of the HAP. In various embodiments, the delay between completion of the administration of the HAP and administration of the second agent is shorter than 8 hours; for example, the delay may be less than 6 or less than 4 hours.

Cancer therapy typically involves multiple cycles of drug administration, and for many cancers, multiple drugs are administered. For illustration, and not for limitation, two anticancer drugs, A and B, may be administered in various administration sequences, as illustrated below:
i. ABAAABAAABAA (repeats or cycles of ABAA);
ii. ABAABAABAABA (cycles of ABA);
iii. ABABABABABABABAB (cycles of AB);
iv. ABBABBABBABBABB (cycles of ABB); and
v. ABBBABBBABBBABBB (cycles of ABBB),
Any of these (and other) cycles of administration can be employed in accordance with the present methods. For example, the sequence ABAAABAAABAA can represent 3 cycles in which "A" is TH-302 administered on Days 1, 8 and 15 of a 28 day cycle, and "B" is gemcitabine administered non-contemporaneously with A on Day 1 of the cycle. It will be understood that where there are multiple cycles and/or multiple administrations a drug(s) within a cycle, the delay between completing administration of HAP and initiation of administration of the non-HAP drug refers to the period between sequential administrations of HAP and non-HAP. For example, in a cycle AB₁B₂B₃ the period between completing administration of A and beginning administration of B₁ is measured (rather than, for example, the period between completing administration of A and beginning administration of B₃).

In one important aspect, described herein are methods of treating pancreatic cancer by administering TH-302 at a dose of either 240 mg/m² or 340 mg/m² in combination with gemcitabine to a patient in need of such treatment. Pancreatic cancer is a malignant neoplasm of the pancreas with current treatment options including surgery, radiotherapy, and chemotherapy. Gemcitabine as a single agent or in combination with other treatments is the most commonly used chemotherapeutic agent in patients with advanced pancreatic cancer. It is estimated that approximately 279,000 cases of pancreatic cancer were diagnosed worldwide in 2008. Pancreatic cancer is the fourth most common cause of cancer death both in the United States and internationally. The American Cancer Society estimates that 44,030 people were diagnosed with pancreatic cancer in the United States in 2011, and approximately 37,660 people died from the disease.

As is discussed below, a wide variety of solid tumors and advanced solid tumors can be treated using such combination therapy, and a wide variety of anticancer agents can be administered in combination with TH-302 for therapeutic benefit. TH-302 may be administered as an initial or first line treatment, for treatment of refractory or metastatic cancer, and as adjuvant or neoadjuvant therapy. In one embodiment of the present invention, the cancer is treated, following diagnosis, in the neoadjuvant setting (chemotherapy is administered to the patient before surgery to shrink the primary tumor and facilitate removal of the primary tumor). In another embodiment, the combination therapy is administered, following diagnosis, as adjuvant treatment (chemotherapy is given after the tumor is removed and the patient is staged; if there is a high likelihood of recurrence then prophylactic chemotherapy is given to delay recurrence and improve survival). In another embodiment, the combination therapy is administered to a patient who has not and is unlikely to have tumor resection surgery. In another embodiment, the combination therapy is administered for treatment of refractory or metastatic cancer (chemotherapy is given for recurrence(s) or spread of the cancer).

Treatable cancers in accordance with the methods herein include, therefore, previously untreated cancers, a refractory cancer, and a metastatic cancer. In one embodiment of the present invention, the relapsed cancer, refractory cancer, or metastatic cancer treated is pancreatic cancer. In other embodiments, the cancer is bile duct cancer, ovarian cancer, esophageal cancer, pancreatic cancer, NSCLC, ampullary cancer, neuroendocrine cancer, soft tissue sarcoma, or thyroid cancer.

For combination therapy, TH-302 (or TH-281) can be administered at frequencies and durations described for above. Thus, in one embodiment of the present invention, the TH-302 is administered at a frequency of once every week. In another embodiment of the present invention, TH-302 is administered in multiple cycles, each cycle of administration being a 4 week cycle, wherein TH-302 is administered once weekly for 3 consecutive weeks. In this embodiment, for each administration cycle, TH-302 administered once weekly for 3 consecutive weeks on days 1, 8, and 15, followed by a week of no TH-302 administration. In one embodiment of the present invention, the TH-302 is administered for a period in the range of 3 weeks to 52 weeks, 3 weeks to 28 weeks, 3 weeks to 16 weeks, and 3 weeks to 8 weeks. Thus, in accordance with the present methods, TH-302 can be administered, for example and without limitation, for 1-13, 1-7, or 1-4 cycles. In some embodiments, certain of these periods of TH-302 administration may include one or more weeks of drug holidays during which no TH-302 is administered.

The formulation and dose, route, frequency, and such other modes of administration of various anticancer agents, other than TH-302, administered in accordance with the present methods, are disclosed herein, are available in medical literature, and/or are known to one of skill in the art. Thus, in some embodiments, the doses of the non-TH-302 chemotherapeutic agent (*e.g.,* gemcitabine) are the approved doses listed in their product labeling. The therapeutically effective amount of an anticancer agent other than TH-302 administered in accordance with the present methods, for anticancer agents approved for use by regulatory authorities, are known to physicians and are provided, for example and without limitation, in the product descriptions found in the PHYSICIANS' DESK REFERENCE, 2003, 57th Ed., Medical Economics Company, Inc., Oradell, N.J; Goodman & Gilman's THE PHARMACOLOGICAL BASIS OF THERAPEUTICS" 2001, 10^{th} Edition, McGraw-Hill, New York; and/or are available from the Federal Drug Administration and/or are discussed in the medical literature. Illustrative dosing amounts and schedules for various cancers in accordance with the methods of the invention are described herein.

For illustration and not for limitation, the following dosages and schedules may be used. Gemcitabine may be administered intravenously (IV) at 1_{.}000 mg/m² over 30 minutes. For example, gemcitabine may be administered IV at 1,000 mg/m² over 30 minutes on Days 1, 8, and 15 of each 28-day cycle and TH-302 may be administered on Days 1, 8, and 15 of each 28-day cycle. In another example, TH-302 followed by gemcitabine are administered once weekly for seven weeks followed by one week of no administration, followed by one or more 28-day cycles in which TH-302 and gemcitabine are administered on Days 1, 8, and 15 of each 28-day cycle.

Therapeutically effective doses of gemcitabine may be determined by medical professionals by reference to materials available from the FDA and/or the medical literature. An exemplary dose is 1000 mg/m² given IV over 30 minutes on days 1, 8 and 15 of a 28 day cycle. Therapeutically effective doses of TH-302 are described above. Other doses may be used as deemed appropriate by medical professionals and/or approved by the FDA.

Described herein is a method for treating a patient diagnosed with pancreatic cancer comprising administering a therapeutically effective dose of TH-302 in combination with administering a therapeutically effective dose of gemcitabine. Administration of TH-302 and gemcitabine provides benefit to patients with first-line pancreatic cancer. A dose of either 240 mg/m² or 340 mg/m² TH-302 is effective for treating subjects with first-line pancreatic cancer.

In some embodiments, administration of gemcitabine is preceded by administration of TH-302, with the gap between the end of TH-302 administration and the beginning of gemcitabine administration being at least 30 minutes to at least one hour, or at least 2 hours, or in some embodiments no more than 24 hours. In some embodiments, the duration of the time between TH-302 and gemcitabine administrations is from 30 minutes to 10 hours, from 2 to 6 hours, or from 3 to 5 hours. In some embodiments the gap between the end of TH-302 administration and the beginning of gemcitabine administration is about 30 minutes to about 2 hours.

### VI. Clinical testing

The products, methods, and technology of the invention may be demonstrated to work in an appropriately designed trial in animal or human subjects.

By way of illustration, a 214 patient randomized controlled Phase 2b clinical trial was conducted to demonstrate the efficacy and safety of TH-302 in the treatment of pancreatic cancer. Administration of 240 mg/m² or 340 mg/m² of TH-302 in combination with gemcitabine was compared with administration of gemcitabine alone as a first-line treatment of in patients with advanced pancreatic cancer. The trial achieved its primary endpoint, with a 63% improvement in progression free survival. Furthermore, secondary efficacy endpoints also trended in favor of the gemcitabine plus TH-302 combination arm compared to the gemcitabine alone control. The trial thus demonstrated that TH-302 confers tangible benefit to patients with aggressive and difficult to treat pancreatic cancer.

The clinical trial was conducted as a multi-center, randomized, controlled, dose-ranging, Phase 2b crossover clinical trial of TH-302 in combination with gemcitabine in patients with first line advanced pancreatic cancer. The primary endpoint of the trial was progression-tree survival. The secondary endpoints were overall response rate, overall survival, change in CA19-9 as well as various other efficacy and safety parameters. Tumor response was evaluated at baseline and every eight weeks using RECIST. Patients for whom monotherapy with gemcitabine is considered standard therapy were eligible for the trial. Patients were randomised equally into one of three cohorts: TH-302 at a dose of 240 mg/m² plus gemcitabine, TH-302 at a dose of 340 mg/m² plus gemcitabine, or gemcitabine alone. Patients who successfully completed six cycles of treatment without evidence of significant treatment-related toxicity or progressive disease could continue to receive treatment. If a patient experienced cancer progression on gemcitabine alone, the patient could cross over and be randomized into one of the TH-302 plus gemcitabine cohorts. The primary efficacy analysis was performed based upon 149 investigator-assessed PFS events and per protocol pooled the data from the two gemcitabine plus TH-302 dose groups in the comparison to gemcitabine alone.

The results of the trial were generally as follows (various results are provided in the attached drawings as well). The median progression-free survival (PFS) was 5.6 months for patients treated with gemcitabine in combination with TH-302 at 240 mg/m² and 6.0 months for patients treated with gemcitabine in combination with TH-302 at 340 mg/m² and 3.6 months for patients treated with gemcitabine alone. The PFS hazard ratio comparing the TH-302 combination to gemcitabine alone was 0.61 (95% confidence interval: 0.43 - 0.87), which was highly statistically significant (p = 0.005). The response rate in the combination arms was 22% compared to 12% in the gemcitabine alone arm, including a response rate of 27% in the gemcitabine plus TH-302 at 340 mg/m² group and 17% in the gemcitabine plus TH-302 at 240 mg/m² group. Other efficacy results also demonstrated the greatest efficacy in the 340 mg/m² group, consistent with a dose-response for TH-302.

The trial demonstrated that the drug combination was well tolerated with a good safety profile. Skin and mucosal toxicities related to TH-302 were dose dependent but not dose limiting. Myelosuppression was dose limiting and generally higher in the combination arms. The trial demonstrated that TH-302 contributes to the efficacy of gemcitabine for the treatment of pancreatic cancer.

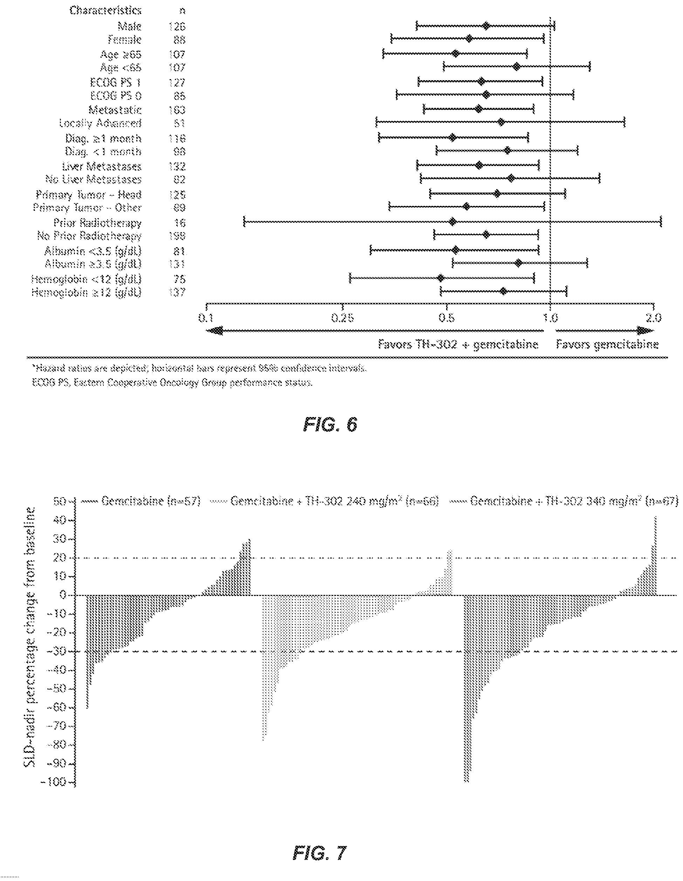

Tumor masses were imaged and the longest diameter was summed for all tumors in each subject (SLD). It was found that the SLD decreased in the treated groups with a median of about 20% over the course of the therapy, and was 30%, 50%, 75%, and 90% reduced in some of the treated groups (**Fig. 7**).

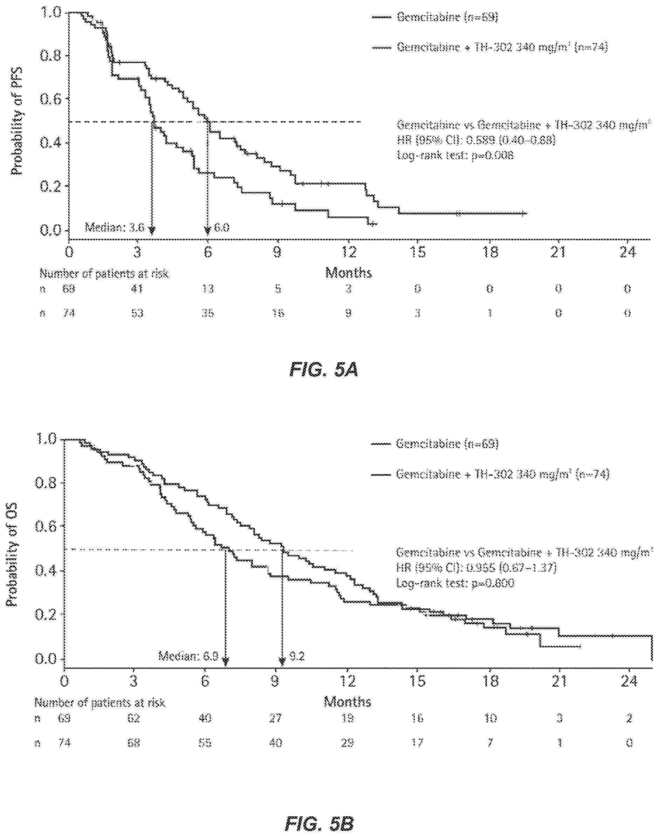

Overall survival (OS) was also monitored. It was found that for pancreatic cancer, a median survival benefit of about 2 to 3 months was achieved over the time period of the study (**Fig. 5B**). Depending on the treated population, the duration of treatment and other factors, the patient treated may attain least two and potentially three, four, or more than four months extended survival.

### VII. Identifying patient subgroups who benefit from treatment

It has been discovered that certain subgroups of cancer patients are particularly suitable for treatment with hypoxia activated prodrugs in accordance with this invention.

Generally, a managing clinician may elect to treat any patient with a hypoxia activated prodrug based upon any criteria deemed to warrant treatment. Such criteria may include type and stage of cancer, characteristics of the subject, histopathology of the tumor, clinical signs, patient and family history, previous therapies given, and treatment objectives in any combination. The following information assists the clinician in determining whether a particular subject or group of subjects (in combination with other considerations) is a good candidate for treatment with a hypoxia activated prodrug in accordance with this invention.

Clinical testing has yielded the following PFS subgroup analysis.

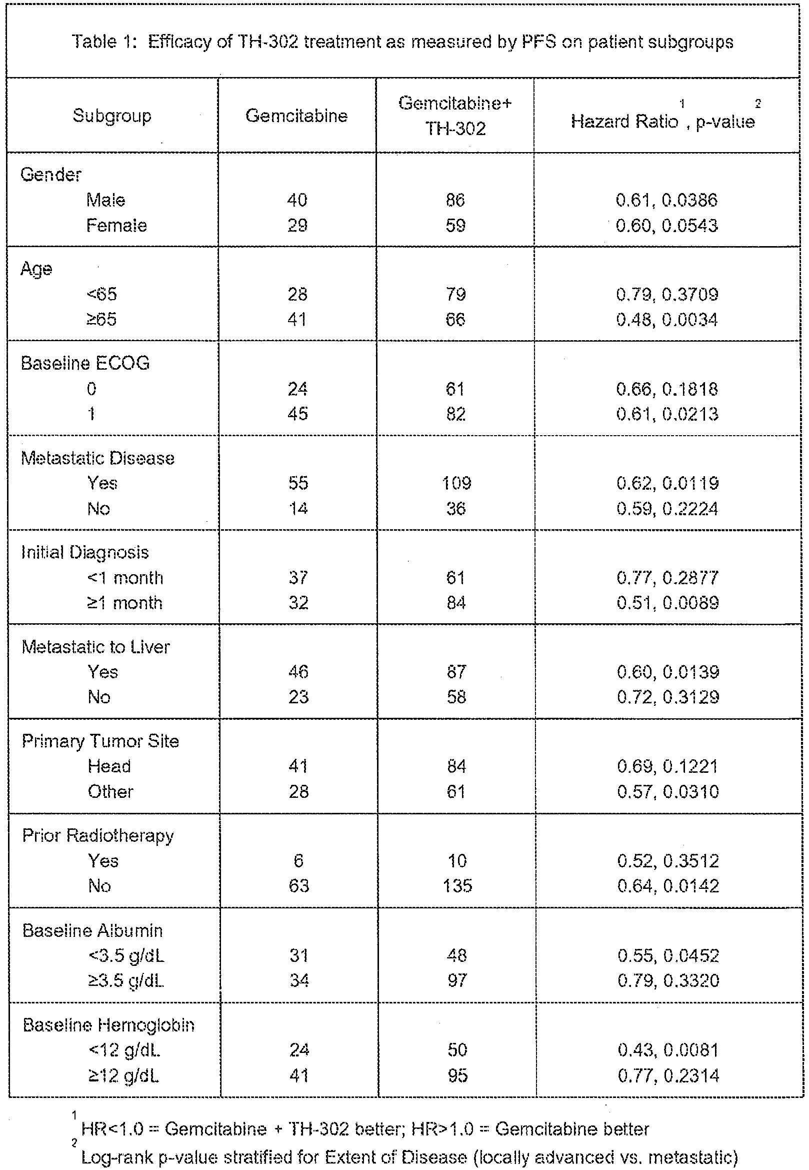

Subjects and subject groups may be selected for treatment according to any or all of the characteristics listed above and elsewhere in this disclosure in any combination, taking into account their clinical status, history, and results of previous treatment. The nature of therapy (such as the dosage and timing of the hypoxia activated prodrug and the other chemotherapy drug) may also be chosen according to any or all of the characteristics listed above and elsewhere in this disclosure in any combination. Furthermore, serum or plasma biomarkers may also be utilized for subject selection. See, e.g., PCT Patent Application No. US2013/023921

Thus, one aspect of this invention is identifying subjects having cancer as eligible or more likely to benefit for treatment of their cancer with a combination of a hypoxia activated prodrug according to Formula I and a chemotherapeutic agent that is not a hypoxia activated prodrug. The method comprises determining whether the subject has one or more of the characteristics listed above, and identifying the subject as eligible for treatment of cancer with said prodrug and chemotherapeutic agent if the subject is determined to have at least one of the characteristics. Cancer may then be selectively treated in patients identified by these criteria.

Factors that predispose towards a favorable result of therapy with TH-302 and related hypoxia activated drugs include the following: The subject is age of 50, 60, 65, 70, 75 or older. The subject is restricted in physically strenuous activity as a result of the cancer. This can be measured on the ECOG performance scale, where 0 means fully active, able to carry on all pre-disease performance without restriction; 1 means restricted in physically strenuous activity but ambulatory and able to carry out light house or office work; 2 means ambulatory and capable of all self-care but unable to carry out any work activities; 3 means capable of only limited self-care, confined to bed or chair more than 50% of waking hours; and 4 means completely disabled. ECOG score of at least 1, 2, 3, or 4 may particularly benefit from the treatment.

Other factors that predispose towards a favorable result of therapy with TH-302 include whether the subject has metastatic cancer. This can be determined by radiologic or magnetic resonance imaging, or by biopsy. The cancer can have metastasized locally or to other sites. Cancer metastasized to the liver is a good candidate for therapy. Another factor is detection of the cancer in the subject at least one, two, three or more than three months before treatment. The subject has not been treated with radiotherapy for the cancer. The treatment may also work better if the subject has not previously failed another form of chemotherapy. The subject has blood markers that are consistent with anemia, cachexia, or inflammation, such as a circulating albumin level below 3.5 g/dL or between 1 and 5 g/dL; or a hemoglobin level below 12 g/dL or a hemoglobin level in the range of 5 to 15 g/dL. Those of skill in the art will recognize that many of the factors that are indicative of a likely favorable response to TH-302 are factors that are otherwise negative prognostic factors. For example, a hemoglobin level below 12 g/dL is indicative of an anemic state; surprisingly, such patients are likely to response favorably to treatment with a hypoxia-activated prodrug such as TH-302 in accordance with the methods of the invention.

Other factors that predispose towards a favorable, ineffective, or adverse outcome will be evident to the reader from other information provided in this disclosure, and may be implemented in this aspect of the invention.

### VIII. Managing side effects

It has been determined that the class of hypoxia activated prodrugs specified in this disclosure may cause particular unwanted side effects or adverse events (AE) in a proportion of subjects treated with a hypoxia activated prodrug, optionally in combination with another chemotherapeutic such as gemcitabine. Such AEs typically do not require the subject to withdraw from treatment. However, the managing clinician is generally advised to be on the lookout for such AEs during treatment, and manage them if they arise. This may improve tolerability of the prodrug, compliance, efficacy of treatment, and quality of life.

Side effects to watch for and manage include clinical signs and/or laboratory results such as rash, nausea, vomiting, stomatitis, skin hyperpigmentation, decreased appetite, febrile neutropenia, pancytopenia, thrombocytopenia, cellulitis, and fatigue. Other signs are pyrexia and diarrhea.

Clinical management of stomatitis may include improving oral health and general care, and any one or more of the following regimens in any combination: (1) rinsing, for example, with a saline or bicarbonate solution; (2) applying one or more coating or lubricating agents or devices such as bismuth salicylate, suralfate, Amphojel^{®}, Kaopectate^{®}, hydroxypropyl methylcellulose film-forming agents such as Zilactin^{®}, and Gelclair^{®}; (3) applying one or more topical analgesics or anesthetics such as benzydamine hydrochloride, lidocaine, dyclonine hydrochloride, diphenhydramine solution, or opioids; (4) administering a growth factor, wound closing agent or healing promoter such as keratinocyte growth factor-1 (KCF-1) available under the trade name Palifermin^{®}.

Management of nausea and vomiting may include dietary adjustment and proper hydration. Drugs that can be used to treat nausea and vomiting include aprepitant, dolasetron, granisetron, ondansetron, palonosetron, proclorperazine, promethazine, lorazepam, metoclopramide, dexamethasone, famotidine, and ranitidine.

Management of anemia (febrile neutropenia, pancytopenia, thrombocytopenia, pancytopenia, thrombocytopenia) may include blood transfusions, and a drug or product that promotes hemogenesis, such as epoetin, darboetin, and oral or intravenous iron supplements

To equip a clinic for carrying out treatment of cancer with hypoxia activated prodrugs and other cancer drugs in accordance with this invention, the manufacturer or distributor has the option of packaging, warehousing, or promoting these drugs in combination. Thus, one aspect (not claimed) is a combination of agents for use in treating cancer, comprising a hypoxia activated prodrug according to Formula 1; optionally a chemotherapeutic agent that is not a hypoxia activated prodrug; and optionally a pharmaceutical agent formulated to reduce one or more side effects(s) induced in a subject by administration of the prodrug and the chernotherapeutic agent.

Thus, the present invention provides a technology for treating cancer and to inhibit abnormal, pathological or unwanted cell growth. The technology includes formulation as a medicament and clinical use of a hypoxia activated prodrug according to Formula I such as TH-302, in combination with other drugs. Such drugs include chemotherapeutic agents such as gemcitabine, and/or drugs to manage unwanted side effects of the TH-302 and/or a coadministered chemotherapeutic agent. The active ingredients of such combinations can be used either simultaneously or sequentially as directed by the managing clinician. Hypoxia activated prodrugs may act synergistically with one or more chemotherapeutic agents in the combinations of this invention to provide an improvement in one or more signs and/or symptoms of the condition being treated that is superior to either agent alone,

Also disclosed herein is a set (kit) consisting of separate packs of an effective amount of a compound according to Formula I, such as TH-302, and/or pharmaceutically acceptable salts, derivatives, solvates and stereoisomers thereof, including mixtures thereof in all ratios, and an effective amount of a further medicament active ingredient. The set comprises suitable containers, such as boxes, individual bottles, bags or ampoules. The set may, for example, comprise separate ampoules, each containing an effective amount of a compound according to the invention and/or pharmaceutically acceptable salts, derivatives, solvates and stereoisomers thereof, including mixtures thereof in all ratios, and an effective amount of a further medicament active ingredient in dissolved or lyophilized form. The kit of the invention may also contain an article that contains written instructions or points the user towards written instructions which explain the handling of the drugs in the kit.

Also disclosed herein is the use of compounds according to Formula I and/or physiologically acceptable salts thereof for the prophylactic or therapeutic treatment and/or monitoring of diseases, such as cancer, that are caused, mediated and/or propagated by undesired cell proliferation. Also disclosed herein is the use of compounds according to Formula I and/or physiologically acceptable salts thereof for the production of a medicament for the prophylactic or therapeutic treatment and/or monitoring of diseases, such as cancer, that are caused, mediated and/or propagated by undesired cell proliferation.

Compounds of Formula I and/or a physiologically acceptable salt thereof can furthermore be employed as intermediates for the preparation of further medicament active ingredients. The medicament may be prepared in a non-chemical manner, e.g. by combining the active ingredient with at least one solid, fluid and/or semi-fluid carrier or excipient, and optionally in conjunction with a single or more other active substances in an appropriate dosage form.

Another aspect is compounds of Formula I according to the invention and/or physiologically acceptable salts thereof for use in the prophylactic or therapeutic treatment and/or monitoring of diseases, such as cancer, that are caused, mediated and/or propagated by undesired cell proliferation. Another aspect concerns compounds of Formula I and/or physiologically acceptable salts thereof for use in the prophylactic or therapeutic treatment and/or monitoring of hyperproliferative disorders. The prior teaching of this specification concerning the compounds of Formula I, including any embodiment thereof, such as TH-302, is valid and applicable without restrictions to other compounds according to Formula I and their salts for use in the prophylactic or therapeutic treatment and/or monitoring of hyperproliferative disorders.

### EXAMPLES

The following examples are intended for illustration only and should not be construed to limit the scope of the invention.

### Example 1

A Phase 1/2, multicenter, dose-escalation study was conducted using a classic dose escalation design followed by a dose expansion to demonstrate the efficacy and determine the safety of TH-302 when administered in combination with gemcitabine. The initial dose of TH-302 was 240 mg/m². TH-302 was administered by intravenous (IV) infusion over 30 minutes on Days 1, 8, and 15 of a 28-day (4 week) cycle (Arm A). Gemcitabine was administered 2 h after the TH-302 infusion was completed. The starting doses of gemcitabine remained fixed according to the approved dose listed in the respective product labeling. The treatment regimen, dose, schedule and cycle length of gemcitabine was as follows. Gemcitabine was administered IV at 1,000 mg/m² IV over 30 minutes on Days 1, 8, and 15 of each 28-day cycle. TH-302 was administered as above on Days 1, 8, and 15 of each 28-day cycle.

The dose was initiated at 240 mg/m² and dose escalation was then continued with 40% increases from the previous dose level; however lower dose increases of 20-40% were also applicable based on treatment outcome. The dose of TH-302 was escalated in cohorts of 3-6 patients. If a subject experienced a dose limiting toxicity (DLT), 3 additional patients were enrolled at that dose level for a total of 6 patients in that cohort. If no additional DLTs were observed, dose escalation was resumed. However, if 2 or more of 6 patients within a cohort experience a DLT, that dose was considered to exceed the maximum tolerated dose (MTD). The MTD was then defined at the next lower dose level in which 6 patients were treated and less than 1 subject experienced a DLT. CT scans were done every 2 cycles in Phase 1.

The objectives of the Phase 1 study were to determine the MTD and DLT of TH-302 and to evaluate the safety, pharmacokinetics (PK) and preliminary efficacy of TH-302 in combination with gemcitabine. The MTD of TH-302 plus gemcitabine was 340 mg/m². The primary dose limiting toxicities were hematologic. While the contribution of TH-302 to the hematologic toxicity is hard to determine when TH-302 is combined with a myelosuppressive chemotherapy, greater hematologic toxicity than would be expected with single agent chemotherapy was evident. Skin and mucosal toxicity were common at doses above 240 mg/m². The mechanism is unknown but may be due to activation of TH-302 in areas of epithelium that are normally hypoxic. The addition of TH-302 to standard chemotherapies does not appear to enhance the toxicity in other body systems. Higher response rates than would be expected with single agent chemotherapy were evident in the Phase 1/2 trial.

### Example 2

In a separate Phase 2b trial, TH-302 was administered intravenously for 30 to 60 minutes on days 1, 8 and 15 of a 28 day cycle at either 240 or 340 mg/m². Gemcitabine was dosed according to its package insert on days 1, 8 and 15 of a 28 day cycle. The 214 patient randomized controlled Phase 2b clinical trial compared the efficacy and safety of these two doses (240 mg/m² and 340 mg/m²) of TH-302 in combination with gemcitabine to gemcitabine alone in patients with first-line advanced pancreatic cancer. The trial achieved its primary endpoint, with a 63% improvement, in progression free survival. Furthermore, secondary efficacy endpoints also trended in favor of the gemcitabine plus Th-302. combination arms compared to the gemcitabine alone treatment arm control. The trial thus demonstrated that TH-302 confers tangible benefit to patients with aggressive and difficult to treat pancreatic cancer.

The clinical trial was conducted as a multi-center, randomized, controlled, dose-ranging, Phase 2b crossover clinical trial of TH-302 in combination with gemcitabine in patients with first line advanced pancreatic cancer. The primary endpoint of the trial was progression-free survival. The secondary endpoints were overall response rate, overall survival, change in CA19-9 as well as various other efficacy and safety parameters. Tumor response was evaluated at baseline and every eight weeks using RECIST. Patients for whom monotherapy with gemcitabine is considered standard therapy were eligible for the trial. Patients were randomized equally into one of three cohorts: TH-302 at a dose of 240 mg/m² plus gemcitabine, TH-302 at a dose of 340 mg/m² plus gemcitabine, or gemcitabine alone. Patients who successfully completed six cycles of treatment without evidence of significant treatment-related toxicity or progressive disease could continue to receive treatment. If a patient experienced cancer progression on gemcitabine alone, the patient could cross over into one of the TH-302 plus gemcitabine cohorts. The primary efficacy analysis was performed based upon 149 investigator-assessed PFS events and per protocol pooled the data from the two gemcitabine plus TH-302 dose groups in the comparison to gemcitabine alone.

The results of the trial were generally as follows (various results are provided in the attached drawings as well). The median progression-free survival (PFS) was 5.6 months for patients treated with gemcitabine in combination with TH-302 at 240 mg/m² and 6 months for patients treated with gemcitabine in combination with TH-302 at 340 mg/m² as compared to 3.6 months for patients treated with gemcitabine alone. The PFS hazard ratio comparing the TH-302 combination to gemcitabine alone was 0.61 (95% confidence interval: 0.43 to 0.87), which was highly statistically significant (p = 0.005). The response rate in the combination arms was 22% compared to 12% in the gemcitabine alone arm, including a response rate of 27% in the gemcitabine plus TH-302 at 340 mg/m² group and 17% in the gemcitabine plus TH-302 at 240 mg/m² group. Other efficacy results also demonstrated the greatest efficacy in the 340 mg/m² group, consistent with a dose-response with TH-302.

The trial demonstrated that the drug combination was well tolerated with a good safety profile. Skin and mucosal toxicities related to TH-302 were dose dependent but not dose limiting. Myelosuppression was dose limiting and generally higher in the combination arms. The trial demonstrated that TH-302 contributes to the efficacy of gemcitabine for the treatment of pancreatic cancer.

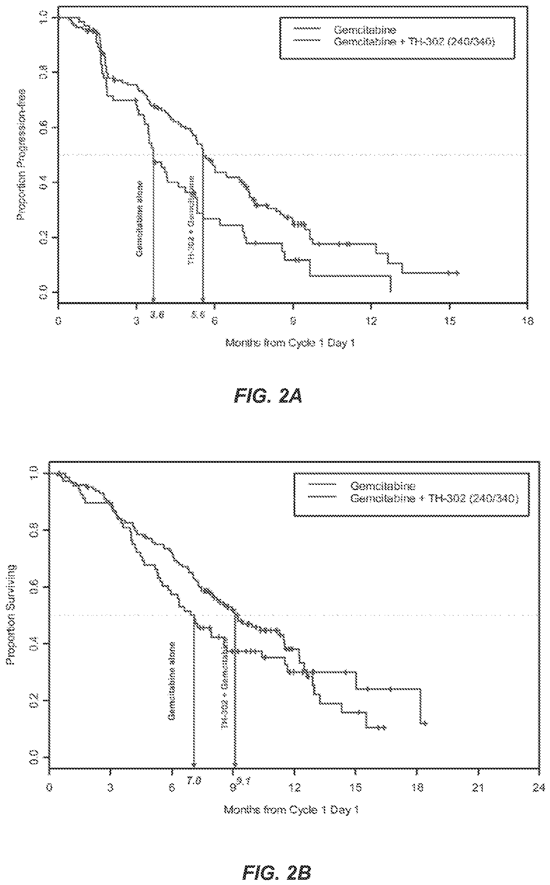

**Fig. 2A** is a Kaplan Meyer curve of the progression free survival (PFS) when the control treated with gemcitabine alone is compared with the two groups (combined) that received the TH-302 at different doses. The median PFS went from 3.6 to 5.6 months: an improvement of about 2 months in the TH-302 treated groups. **Fig. 2B** shows that in parallel, the overall survival (OS) improved by about the same amount.

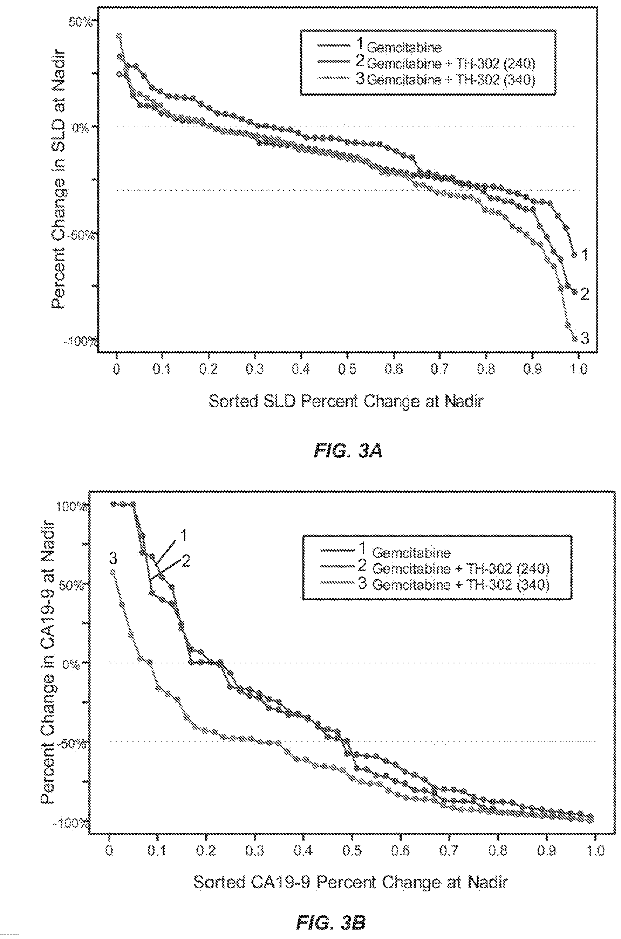

**Fig. 3A** is a waterfall plot showing the decrease in tumor size in the three groups. Tumor size is calculated as the sum of longest diameters of all tumors in each subject from a baseline established before treatment. The groups receiving TH-302 followed by gemcitabine showed more tumor shrinkage. The effect was dependent on the dose of the TH-302 given. **Fig. 3B** is a waterfall plot showing the change in the tumor marker CA19-9 in blood of subjects in the three groups. The group showing the greatest decrease had received gemcitabine in combination with TH-302 at 340 mg/m².

### Example 3

This example is an update of the open-label, multicenter, randomized, Phase 2b study (NCT01144455) of two dose levels of TH-302 in combination with gemcitabine vs gemcitabine alone. The update was conducted in September 2012 and including a further follow-up of the data provided in Example 2.

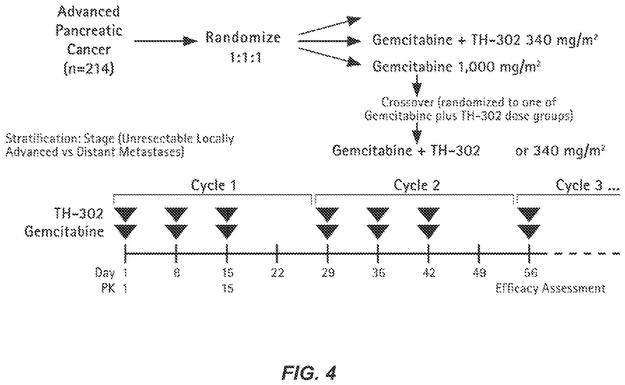

Patients were stratified according to their disease stage (unresectable locally advanced vs distant metastases) and randomized as shown in **Fig. 4**. The treatments were administered intravenously in sequential 30 to 60-minute infusions (TH-302 followed by gemcitabine) on days 1, 8, and 15 of a 28-day cycle. Unless patients experienced progressive disease (PD) or unacceptable toxicity, they could continue to receive treatment beyond 6 cycles if considered clinically beneficial. Patients initially randomized to G alone could cross-over after PD and be randomized to one of the two G+T arms.

Key eligibility criteria included histologically or cytologically confirmed locally advanced or metastatic pancreatic ductal adenocarcinoma, measurable disease according to Response Evaluation Criteria in Solid Tumors (RECIST) version 1.1, an Eastern Cooperative Oncology Group (ECOG) performance status of 0 or 1, and a bilirubin level of ≤ 1.5 times upper limit of normal.

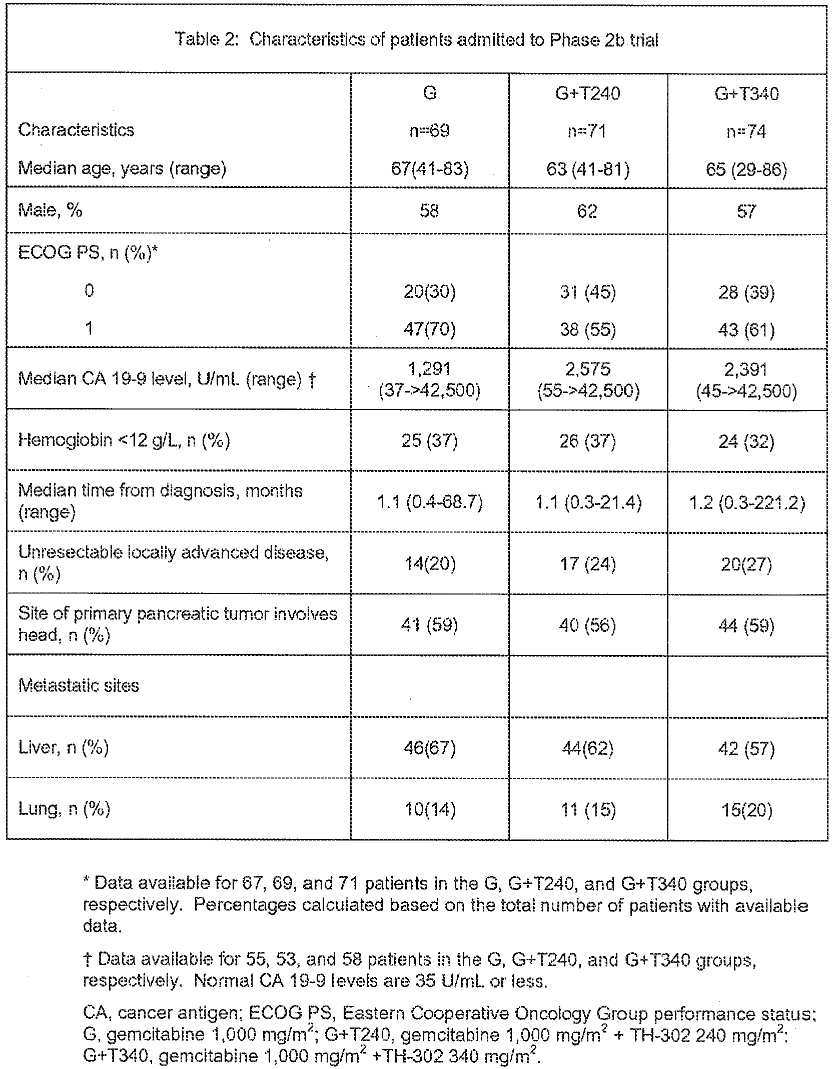

Outcome measures were Progression Free Survival (PFS) and Overall Survival (OS). Both were calculated from day 1 of cycle 1 and assessed using Kaplan-Meier methodology.

A total of 214 patients were treated. Median age was 65 years (range: 29-86) and 126 (59%) patients were male. ECOG performance status was 0 in 38% of patients and 1 in 62% of patients. Overall, 76% and 24% of patients had Stage IV and Stage IIIB disease at baseline, respectively. 32, 45, and 55% of patients in the G, G+T240, and G+T340 arms, respectively, received at least 6 treatment cycles.

### Efficacy

Median PFS was significantly prolonged in the G+T arms vs the G arm (**Fig. 5A**); 5.6 months with G+T240 [p=0.060] and 6.0 months with G+T340 [p=0.008] vs 3.6 months with G). A subgroup analysis of PFS according to patients baseline demographics and disease characteristics favored gemcitabine combined with TH-302 over gemcitabine alone for all analyzed variahlesshown in **Fig. 6****.** CA19-9 responses according to treatment arms are shown in the following Table.

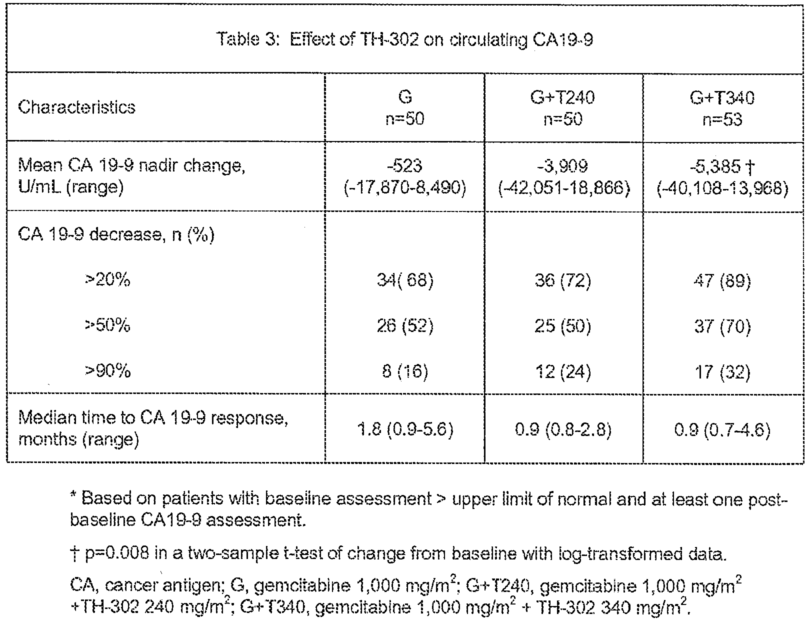

Objective best (unconfirmed) response (complete response [CR] plus partial response [PR]) was observed in 10% of patients in the G arm vs 17% and 26% in the G+T240 and G+T340 arms, respectively (p=0.220 for G+T240 and p=0.021 for G+T340 vs G). Fig. 7 shows a waterfall plot of the tumor shrinkage observed. Disease control (CR, PR, plus stable disease) was observed in 67, 75, and 76% of patients treated with G, G+T240, and G+T340, respectively.

Median OS for G, G+T240, and G+T340 was 6.9, 8.7, and 9.2 months, respectively (Fig. 5B). The 6-month survival rate was 57% in the G arm, 69% in the G+T240 arm (p=0.123) and 73% in the G+T340 arm(p=0.037). The 12-month survival rate was 26% in the G arm, 37% in the G+T240 arm (p=0.178) and 38% in the G+T340 arm (p=0.130). Each of the 6 month and 12 month survival rates of the combination arms was statistically significantly higher (p < 0.20 significance in the Phase 2b study) than the survival in the gemcitabine arm. After 12 months the survival curves crossed, with the contributions of patients receiving combination therapy after crossing over from gemcitabine confounding the longterm survival results.

A total of 14 and 12 patients who were initially randomized to G crossed over to G+T240 and G+T340, respectively. Median post-crossover PFS was 1.8 months with G+T240 vs 2.9 months with G+T340 (p=0.13). Median post-crossover OS was 2.6 months with G+T240 vs 13.4 months with G+T340 (p=0.010).

In conclusion, PFS, the primary study endpoint, was significantly improved in patients treated with the combination of gemcitabine plus TH-302 compared with gemcitabine alone. A consistent dose effect was evident in terms of improved PFS, increased objective response rate, and decreased CA 19-9 levels in the G+T340 arm compared with the G+T240 and the gemcitabine-alone arms.

There was a significant improvement in overall survival at 6 months and at 12 months in both the G+T240 arm and the G+T340 arm compared with the G arm. The overall survival curves were not not significantly different across treatment aims. The results can be partially explained by control arm patients with PD crossing over to one of the G+T treatment arms. There was significant longer survival with G+T340 than with G+T240 following crossover after progression on the G arm.

### Side effects

Non-laboratory adverse events (AEs) are reported in the table below. Rash and stomatitis occurred more frequently in the G+T arms. However, only 3 patients experienced Grade 3 rash and all reported events of stomatitis were of Grade 1/2. Treatment-emergent Grade 3/4 thrombocytopenia was reported in 10, 28, and 57% in the G, G+T240, and G+T340 arms, respectively. No patients discontinued study treatment due to bleeding as a consequence of low platelet counts.

Treatment-emergent Grade 3/4 neutropenia was observed in 16, 32, and 42% of patients treated with G, G+T240, and G+T340, respectively, and 0, 1.4 and 5.4% of patients, respectively, experienced Grade 3/4 febrile neutropenia. No patients discontinued the study because of febrile neutropenia.

Skin or mucosal toxicity and myelosuppression were the most commonly reported TH-302-related AEs. These AEs were manageable. There was no increase in treatment discontinuation compared with gemcitabine alone.

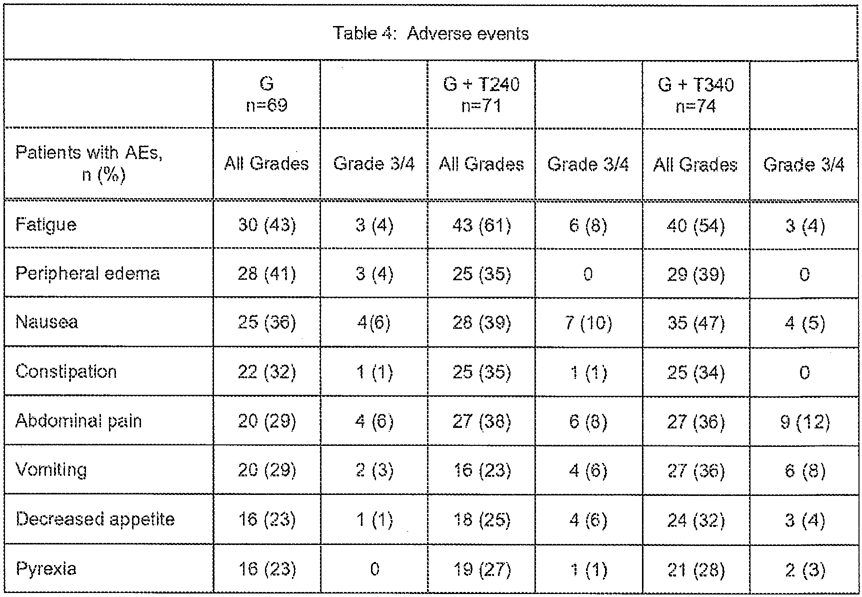

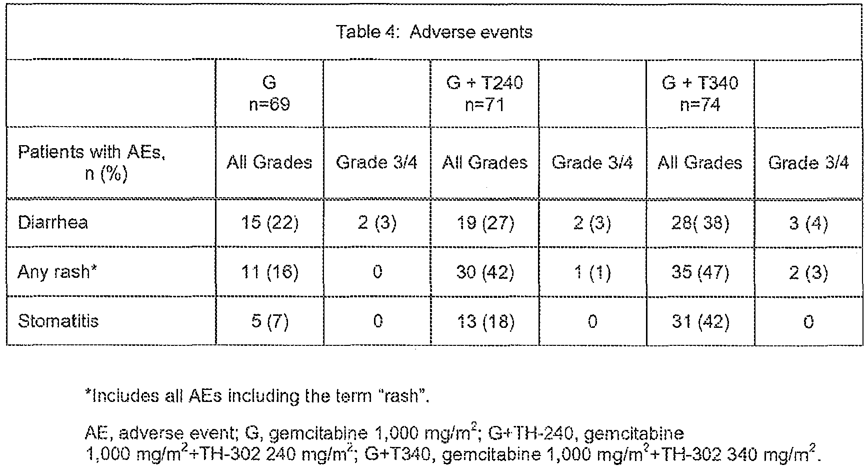

## Claims

1. A method of identifying a subject as eligible for treatment of its cancer with a combination of a hypoxia activated prodrug TH-302 or TH-281 or a pharmaceutically acceptable salt thereof;
in combination with a chemotherapeutic agent that is a nucleoside analog; comprising:
(a) determining whether the subject has the following characteristics:
(i) the subject has an ECOG performance status of at least 2, 3, or 4; and
(ii) the subject has a circulating albumin level below 3.5 g/dL;
(b) identifying the subject as eligible for treatment of their cancer with said prodrug and chemotherapeutic agent if they are determined to have the listed characteristics in step (a).

2. A combination of a hypoxia activated prodrug TH-302 or TH-281 or a pharmaceutically acceptable salt thereof;
and a chemotherapeutic agent that is a nucleoside analog for use in the treatment of cancer in a subject having the following characteristics:
(i) the subject has an ECOG performance status of at least 2, 3, or 4; and
(ii) the subject has a circulating albumin level below 3.5 g/dL.

3. The combination for use of claim 2, further comprising a pharmaceutical agent formulated to reduce the side effects(s) of the hypoxia activated prodrug in the subject; wherein the side effects are selected from fatigue, peripheral edema, nausea, constipation, abdominal pain, vomiting, decreased appetite, pyrexia, diarrhea, rash or stomatitis.

4. The method according to claim 1, wherein the cancer is pancreatic cancer.

5. The method according to claim 4, wherein the cancer is pancreatic ductal adenocarcinoma.

6. The combination for use according to claim 2 or 3, wherein the cancer is pancreatic cancer.

7. The combination for use according to claim 6, wherein the cancer is pancreatic ductal adenocarcinoma.

8. The combination for use according to claim 6, wherein the mass of one or more tumors in subjects having pancreatic ductal adenocarcinoma is reduced such that that the median percentage change in sum of longest diameter (SLD) from baseline to nadir in said tumor mass is reduced by at least 20%.

9. The combination for use according to claim 6, wherein the overall survival in a patient having pancreatic ductal adenocarcinoma is increased, whereby the median overall survival of a patient treated according to the method is increased by at least two months, as compared to the overall survival said patient would be estimated to have with treatment with the chemotherapeutic agent alone.

10. The method according to any one of claims 1, 4, or 5, wherein the hypoxia activated prodrug is TH-302.

11. The combination for use according to any of claims 2, 3 or 6 to 9, wherein the hypoxia activated prodrug is TH-302.

12. The combination for use according to claim 11, wherein the TH-302 is administered intravenously in an amount of either 240 mg/m² or 340 mg/m² in each cycle of treatment.

13. The combination for use according to claim 11 or 12, wherein each cycle of treatment comprises administration of TH-302, followed by administration of the chemotherapeutic agent 30 minutes to 8 hours after administration of TH-302 is completed.

14. The method according to any one of claims 1, 4, 5, or 10 or the combination for use according to any of 2, 3 or 6 to 13, wherein the chemotherapeutic agent is gemcitabine.

15. The method according to any one of claims 1, 4, 5, 10, or 14 or the combination for use according to any one of claims 2, 3, or 6 to 13, wherein the subject has not been treated with radiotherapy for the cancer.

## Patentansprüche

1. Verfahren zur Identifizierung eines Subjekts, das geeignet ist für eine Behandlung seines Krebses mit einer Kombination aus der Hypoxie-aktivierten Prodrug TH-302 oder TH-281 oder eines pharmazeutisch geeigneten Salzes davon;
in Kombination mit einem chemotherapeutischen Wirkstoff, der ein Nukleosidanalogon ist; umfassend:
(a) Bestimmen, ob das Subjekt die folgenden Merkmale aufweist:
(i) das Subjekt hat einen ECOG-Leistungsstatus von mindestens 2, 3 oder 4; und
(ii) das Subjekt hat einen zirkulierenden Albuminspiegel unter 3,5 g/dL;
(b) Identifizierung des Subjekts als geeignet für die Behandlung seines Krebses mit der Prodrug und dem chemotherapeutischen Wirkstoff, wenn bestimmt wurde, dass es die in Schritt (a) aufgeführten Merkmale aufweist.

2. Kombination der Hypoxie-aktivierten Prodrug TH-302 oder TH-281 oder eines pharmazeutisch geeigneten Salzes davon;
und einem chemotherapeutischen Wirkstoff, der ein Nukleosidanalogon ist, zur Verwendung in der Behandlung von Krebs in einem Subjekt, das die folgenden Merkmale hat:
(i) das Subjekt hat einen ECOG-Leistungsstatus von mindestens 2, 3 oder 4; und
(ii) das Subjekt hat einen zirkulierenden Albuminspiegel unter 3,5 g/dL.

3. Kombination zur Verwendung nach Anspruch 2, außerdem umfassend einen pharmazeutischen Wirkstoff, der formuliert ist Nebenwirkung(en) der Hypoxie-aktivierten Prodrug in dem Subjekt zu reduzieren; wobei die Nebenwirkungen ausgewählt sind aus Müdigkeit, peripherem Ödem, Übelkeit, Verstopfung, Unterleibsschmerzen, Erbrechen, vermindertem Appetit, Fieber, Durchfall, Ausschlag oder Stomatitis.

4. Verfahren nach Anspruch 1, wobei der Krebs Bauchspeicheldrüsenkrebs ist.

5. Verfahren nach Anspruch 4, wobei der Krebs ein duktales Adenokarzinom der Bauchspeicheldrüse ist.

6. Kombination zur Verwendung nach Anspruch 2 oder 3, wobei der Krebs Bauchspeicheldrüsenkrebs ist.

7. Kombination zur Verwendung nach Anspruch 6, wobei der Krebs ein duktales Adenokarzinom der Bauchspeicheldrüse ist.

8. Kombination zur Verwendung nach Anspruch 6, wobei die Masse eines oder mehrerer Tumore in Subjekten mit duktalem Adenokarzinom der Bauchspeicheldrüse so reduziert wird, dass die mediane prozentuale Veränderung der Summe des längsten Durchmessers (SLD) von der Grundlinie bis zum Nadir in der Tumormasse um mindestens 20 % reduziert ist.

9. Kombination zur Verwendung nach Anspruch 6, wobei die Gesamtüberlebenszeit bei einem Patienten mit duktalem Adenokarzinom der Bauchspeicheldrüse verlängert ist, wobei die mediane Gesamtüberlebenszeit eines nach dem Verfahren behandelten Patienten um mindestens zwei Monate verlängert ist, verglichen mit der Gesamtüberlebenszeit, die der Patient geschätzt mit der Behandlung mit dem chemotherapeutischen Wirkstoff allein hätte.

10. Verfahren nach einem der Ansprüche 1, 4 oder 5, wobei die Hypoxie-aktivierte Prodrug TH-302 ist.

11. Kombination zur Verwendung nach einem der Ansprüche 2, 3 oder 6 bis 9, wobei die Hypoxie-aktivierte Prodrug TH-302 ist.

12. Kombination zur Verwendung nach Anspruch 11, wobei TH-302 intravenös in einer Menge von entweder 240 mg/m² oder 340 mg/m² in jedem Behandlungszyklus verabreicht wird.

13. Kombination zur Verwendung nach Anspruch 11 oder 12, wobei jeder Behandlungszyklus die Verabreichung von TH-302 umfasst, gefolgt von der Verabreichung des chemotherapeutischen Wirkstoffs 30 Minuten bis 8 Stunden nach Verabreichung von TH-302.

14. Verfahren nach einem der Ansprüche 1, 4, 5 oder 10 oder die Kombination zur Verwendung nach einem der Ansprüche 2, 3 oder 6 bis 13, wobei der chemotherapeutische Wirkstoff Gemcitabin ist.

15. Verfahren nach einem der Ansprüche 1, 4, 5, 10 oder 14 oder die Kombination zur Verwendung nach einem der Ansprüche 2, 3 oder 6 bis 13, wobei das Subjekt nicht mit einer Strahlentherapie gegen den Krebs behandelt wurde.

## Revendications

1. Procédé d'identification d'un sujet comme étant éligible au traitement de son cancer avec une combinaison d'un promédicament activé par l'hypoxie, TH-302 ou TH-281, ou un sel pharmaceutiquement acceptable de celui-ci ;
en combinaison avec un agent chimiothérapeutique qui est un analogue de nucléoside ; comprenant :
(a) déterminer si le sujet présente ou non les caractéristiques suivantes :
(i) le sujet présente un indice de performance ECOG d'au moins 2, 3 ou 4 ; et
(ii) le sujet présente un taux d'albumine circulante inférieur à 3,5 g/dL ;
(b) identifier le sujet comme étant éligible pour le traitement de son cancer avec lesdits promédicament et agent chimiothérapeutique s'il a été déterminé qu'il présente les caractéristiques listées à l'étape (a).

2. Combinaison d'un promédicament activé par l'hypoxie, TH-302 ou TH-281, ou un sel pharmaceutiquement acceptable de celui-ci ;
et d'un agent chimiothérapeutique qui est un analogue de nucléoside pour son utilisation dans le traitement du cancer chez un sujet présentant les caractéristiques suivantes :
(i) le sujet présente un indice de performance ECOG d'au moins 2, 3 ou 4 ; et
(ii) le sujet présente un taux d'albumine circulante inférieur à 3,5 g/dL.

3. Combinaison pour son utilisation selon la revendication 2, comprenant en outre un agent pharmaceutique formulé pour réduire les effets secondaires du promédicament activé par l'hypoxie chez le sujet ; dans laquelle les effets secondaires sont choisis parmi fatigue, œdème périphérique, nausée, constipation, douleurs abdominales, vomissements, diminution de l'appétit, fièvre, diarrhée, éruption cutanée ou stomatite.

4. Procédé selon la revendication 1, dans lequel le cancer est un cancer du pancréas.

5. Procédé selon la revendication 4, dans lequel le cancer est un adénocarcinome canalaire pancréatique.

6. Combinaison pour son utilisation selon la revendication 2 ou 3, dans laquelle le cancer est un cancer du pancréas.

7. Combinaison pour son utilisation selon la revendication 6, dans laquelle le cancer est un adénocarcinome canalaire pancréatique.

8. Combinaison pour son utilisation selon la revendication 6, dans laquelle la masse d'une ou plusieurs tumeurs chez des sujets ayant un adénocarcinome canalaire pancréatique est réduite de telle sorte que le pourcentage de modification médian de la somme des plus grands diamètres (SLD) entre ligne de base et nadir dans ladite masse tumorale est réduit d'au moins 20 %.

9. Combinaison pour son utilisation selon la revendication 6, dans laquelle la survie globale chez un patient ayant un adénocarcinome canalaire pancréatique est augmentée, selon laquelle la survie globale médiane d'un patient traité selon le procédé est augmentée d'au moins 2 mois, en comparaison de l'estimation de la survie globale dudit patient sous traitement avec l'agent chimiothérapeutique seul.

10. Procédé selon l'une quelconque des revendications 1, 4 ou 5, dans lequel le promédicament activé par l'hypoxie est TH-302.

11. Combinaison pour son utilisation selon l'une quelconque des revendications 2, 3 ou 6 à 9, dans laquelle le promédicament activé par l'hypoxie est TH-302.

12. Combinaison pour son utilisation selon la revendication 11, dans laquelle le TH-302 est administré par voie intraveineuse en une quantité de 240 mg/m² ou de 340 mg/m² dans chaque cycle de traitement.

13. Combinaison pour son utilisation selon la revendication 11 ou 12, dans laquelle chaque cycle de traitement comprend l'administration de TH-302 suivie par l'administration de l'agent chimiothérapeutique 30 minutes à 8 heures après l'administration de TH-302.

14. Procédé selon l'une quelconque des revendications 1, 4, 5 ou 10 ou combinaison pour son utilisation selon l'une quelconque des revendications 2, 3 ou 6 à 13, dans lequel l'agent chimiothérapeutique est la gemcitabine.

15. Procédé selon l'une quelconque des revendications 1, 4, 5, 10 ou 14 ou combinaison pour son utilisation selon l'une quelconque des revendications 2, 3 ou 6 à 13, dans lequel le sujet n'a pas été traité par radiothérapie pour le cancer.
